(19) Europäisches Patentamt
European Patent Office
Office européen des brevets

(11) Veröffentlichungsnummer: **0 338 465 B1**

(12) # EUROPÄISCHE PATENTSCHRIFT

(45) Veröffentlichungstag der Patentschrift: **02.06.93**

(51) Int. Cl.[5]: **C07D 403/04**, C07D 409/14, A01N 43/653

(21) Anmeldenummer: **89106777.9**

(22) Anmeldetag: **15.04.89**

(54) **1-Chlorpyrimidinyl-1H-1,2,4-triazol-3-sulfonsäureamide, Verfahren zu ihrer Herstellung und ihre Verwendung als Mittel mit herbizider Wirkung.**

(30) Priorität: **20.04.88 DE 3813885**

(43) Veröffentlichungstag der Anmeldung:
**25.10.89 Patentblatt 89/43**

(45) Bekanntmachung des Hinweises auf die Patenterteilung:
**02.06.93 Patentblatt 93/22**

(84) Benannte Vertragsstaaten:
**AT BE CH DE ES FR GB GR IT LI LU NL SE**

(56) Entgegenhaltungen:
**EP-A- 0 246 749**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Wegner, Peter, Dr.**
**Am Eichenhain 60**
**W-1000 Berlin 28(DE)**
Erfinder: **Krüger, Martin, Dr.**
**Schluchseestrasse 65**
**W-1000 Berlin 28(DE)**
Erfinder: **Johann, Gerhard, Dr.**
**Hermsdorfer Damm 147**
**W-1000 Berlin 28(DE)**
Erfinder: **Rusch, Reinhart, Dr.**
**Wildkanzelweg 29**
**W-1000 Berlin 28(DE)**
Erfinder: **Rees, Richard, Dr.**
**Speerweg 8**
**W-1000 Berlin 28(DE)**
Erfinder: **Head, John, Dr.**
**57, Norfolk Way**
**Bishop's Stortford Herts(GB)**
Erfinder: **Rowson, Graham, Dr.**
**1 Rogeron Close**
**Hundon, Suffolk(GB)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft neue 1-Chlorpyrimidinyl-1H-1,2,4-triazol-3-sulfonsäureamide, Verfahren zu ihrer Herstellung und ihre Verwendung als Mittel mit herbizider Wirkung.

In der EP-Anmeldung 0 246 749 werden herbizid wirksame Triazolsulfonamide der Formel

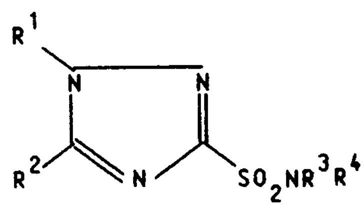

und deren Salze beansprucht, in der

- $R^1$ Wasserstoff, ein substituiertes oder unsubstituiertes Alkyl, Alkenyl, Alkinyl, Cycloalkyl, Aryl, Aralkyl, Acyl, Alkoxycarbonyl, Aminocarbonyl oder Sulfonyl oder eine heterocyclische Gruppe,
- $R^2$ Wasserstoff, Halogen, Cyano, Hydroxy, Mercapto, ein substituiertes oder unsubstituiertes Alkyl, Alkenyl, Alkinyl, Alkoxy, Alkylthio, Alkylsulfinyl, Alkylsulfonyl, Acyl, Alkoxycarbonyl, Aminocarbonyl oder Amino oder eine heterocyclische Gruppe,
- $R^3$ eine substituierte oder unsubstituierte heterocyclische, benzoheterocyclische, Aryl- oder Aralkyl-Gruppe und
- $R^4$ Wasserstoff, ein substituiertes oder unsubstituiertes Alkyl, Alkenyl, Alkinyl, Acyl, Alkylsulfonyl, Alkoxycarbonyl, Aminocarbonyl oder Aralkyl oder eine Gruppe der Formel

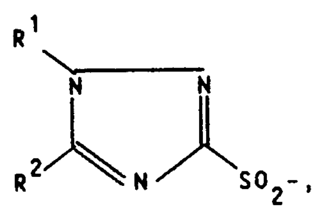

in der $R^1$ und $R^2$ die vorstehend genannten Bedeutungen haben,

darstellen.

Wir haben nun gefunden, daß eine spezielle Gruppe von Verbindungen innerhalb dieses breiten Patentanspruches besonders wertvolle Eigenschaften besitzt. Die Auswahl dieser Gruppe war weder aus der früheren Veröffentlichung voraussehbar, noch konnte vorhergesehen werden, daß diese Gruppe von Verbindungen besondere Vorteile zeigen würde.

Gemäß der Erfindung werden bevorzugt 1-Chlorpyrimidinyl-1H-1,2,4-triazol-3-sulfonsäureamide der allgemeinen Formel I

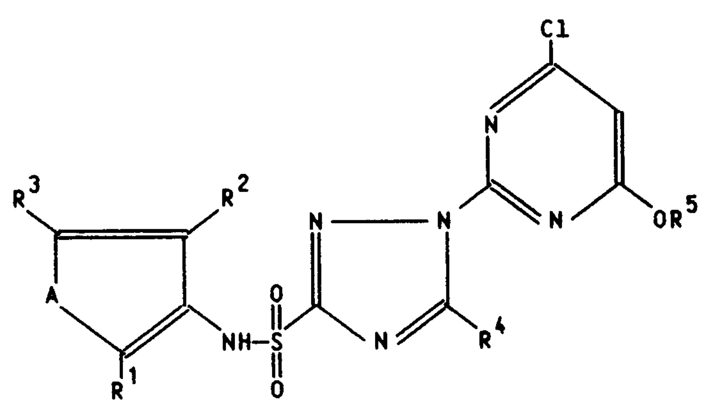

(I) ,

in der

A -S- oder -CH=CH-

$R^1$ Halogen, Methyl, Trifluormethyl, Methoxy, Difluormethoxy, Nitro oder Methoxycarbonyl,

$R^2$ Wasserstoff, Halogen, Methyl, Methoxy, Allyloxy oder Propargyloxy,

$R^3$ Wasserstoff oder Methyl,

$R^4$ Wasserstoff, Methyl oder Methoxymethyl und

$R^5$ $C_1$-$C_4$-Alkyl

bedeuten.

Die erfindungsgemäßen Verbindungen sind somit 1H-1,2,4-Triazol-3-sulfonsäureamidderivate, die an der Sulfonsäureamidbindung eine Phenyl- oder Thienylgruppe (mit festgelegten Substituenten) tragen und bei denen das Triazol in 1-Stellung durch eine 4-Chlor-6-($C_1$-$C_4$-alkoxy)-pyrimidin-2-yl-gruppe und in 5-Stellung durch Wasserstoff, Methyl oder Methoxymethyl substituiert ist. In der EP-Anmeldung 0 246 749 befinden sich keine Beispiele von irgendwelchen Verbindungen, die eine 1-(4-Chlor-6-($C_1$-$C_4$-alkoxy)-pyrimidin-2-yl)-gruppe enthalten. Weiterhin hat das einzige Beispiel einer Verbindung, die eine Thienylgruppe an der Sulfonsäureamidbindung besitzt, keinen Substituenten in der 1-Stellung der Triazolgruppe und ist in 5-Stellung mit einer Pyrrolgruppe substituiert.

Die erfindungsgemäßen Verbindungen sind gekennzeichnet durch eine gute herbizide Wirkung, insbesondere durch eine hohe Wirksamkeit gegen Matricaria spp., verbunden mit einer guten Nutzpflanzenselektivität, insbesondere in Zuckerrüben. Diese Kombination einer guten Wirksamkeit gegen Matricaria (die ein besonders wichtiges und schwierig zu bekämpfendes Unkraut in Zuckerrüben darstellt) mit einer guten Selektivität in Zuckerrüben ist bei den Verbindungen, die in der EP-Anmeldung 0 246 749 genannt sind, nicht erkennbar.

Die erfindungsgemäßen Verbindungen der allgemeinen Formel I können zum Beispiel hergestellt werden, indem man

A) Amine der allgemeinen Formel II

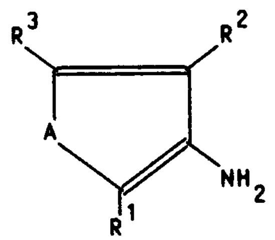

(II),

in der A, $R^1$, $R^2$ und $R^3$ die oben angegebenen Bedeutungen haben, mit einem Sulfonsäurechlorid der allgemeinen Formel III

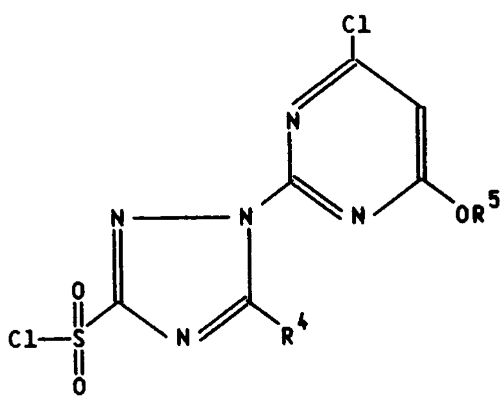

(III),

in der $R^4$ und $R^5$ die oben angegebenen Bedeutungen haben, in einem geeigneten Lösungsmittel in Gegenwart eines Säureakzeptors umsetzt, oder

B) eine Verbindung der allgemeinen Formel IV

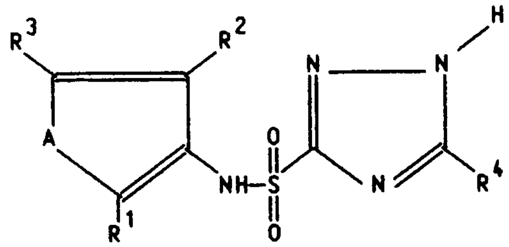

(IV),

in der A, $R^1$, $R^2$, $R^3$ und $R^4$ die oben genannten Bedeutungen haben, mit einer Verbindung der allgemeinen Formel V

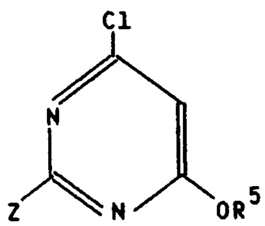

(V) ,

in der Z für Chlor, Brom oder eine Alkyl- oder Arylsulfonylgruppe steht und $R^5$ die oben genannte Bedeutung hat, in einem geeigneten Lösungsmittel in Gegenwart eines säurebindenden Mittels umsetzt.

Die einzelnen Verfahrensvarianten werden vorzugsweise in Gegenwart eines Verdünnungsmittels durchgeführt. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie Beispiel Dimethylformamid und Dimethylacetamid, Sulfone und Sulfoxide, wie Beispiel Dimethylsulfoxid und Sulfolan, und Basen, wie zum Beispiel Pyridin.

Die Reaktionen werden vorzugsweise zwischen Raumtemperatur und dem Siedepunkt des jeweiligen Reaktionsgemisches durchgeführt. Die Reaktionen lassen sich bei dem Druck der Umgebung durchführen, wenngleich sie auch bei erhöhtem beziehungsweise vermindertem Druck durchgeführt werden können.

Die Verfahrensvariante A) wird vorzugsweise in chlorierten Kohlenwasserstoffen, wie Dichlormethan oder Dichlorethan, in Anwesenheit eines Katalysators beziehungsweise Säureakzeptors durchgeführt. Beispiele hierfür sind tertiäre Amine, wie zum Beispiel Triethylamin, Diisopropylethylamin, N-Methylmorpholin, 4-Dimethylaminopyridin und Pyridin. Pyridin kann sowohl als Katalysator als auch als Lösungsmittel bei dieser Reaktion verwendet werden.

Die Verfahrensvariante B) wird bevorzugt in inerten Lösungsmitteln, wie Dioxan, Tetrahydrofuran, Dimethylformamid oder N-Methylpyrrolidinon, gegebenenfalls in Anwesenheit eines Katalysators durchgeführt. Beispiele für säurebindende Mittel sind Metallhydride, tert. Amine, wie zum Beispiel Triethylamin oder Diisopropylethylamin und anorganische Basen, wie zum Beispiel Alkali- oder Erdalkalihydroxide odercarbonate.

Die nach den oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel farb- und geruchlose Kristalle dar, die wenig löslich in Wasser und in aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, und Sulfoxiden, wie Dimethylsulfoxid, sind.

Die Verbindungen der allgemeinen Formeln II, III, IV und V können nach in der Literatur beschriebenen Verfahren hergestellt werden, insbesondere nach den in der EP-Anmeldung 0 246 749 genannten Verfahren.

Wie bereits weiter oben ausgeführt, zeigen die erfindungsgemäßen Verbindungen eine gute Herbizidwirkung. Diese Wirkung erstreckt sich auf monokotyle und dikotyle Unkräuter bei einer guten Selektivität in verschiedenen Nutzpflanzen, wie zum Beispiel in Zuckerrüben.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je nach Anwendungsart im Vor- und Hachauflauf in Grenzen zwischen 0,01 bis 5 kg/ha.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden.

Beispielsweise eignen sich als herbizid wirksame Mischungspartner diejenigen Wirkstoffe, die in Weed Abstracts, Vol. 36, No. 12, 1987, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahrenist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.)
25 Gewichtsprozent Wirkstoff
60 Gewichtsprozent Kaolin
10 Gewichtsprozent Kieselsäure
5 Gewichtsprozent einer Mischung aus dem Calciumsalz der Ligninsulfonsäure und dem Natriumsalz des N-Methyl-N-oleyl-taurins
2.)
40 Gewichtsprozent Wirkstoff
25 Gewichtsprozent Tonmineralien
25 Gewichtsprozent Kieselsäure
10 Gewichtsprozent einer Mischung aus dem Calciumsalz der Ligninsulfonsäure und Alkylphenylpolyglycolethern

B) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

C) **Emulsionskonzentrat**

25 Gewichtsprozent Wirkstoff
15 Gewichtsprozent Cyclohexanon
55 Gewichtsprozent Xylol
5 Gewichtsprozent einer Mischung aus dem Calciumsalz der Dodecylbenzolsulfonsäure und Nonylphenylpolyoxyethylen

Die nachstehenden Beispiele erläutern die Herstellung der erfindungsgemäßen Verbindungen:

Beispiel 1

1-(4-Chlor-6-methoxypyrimidin-2-yl)-1H-1,2,4-triazol-3-sulfonsäure-(2-difluormethoxy-6-methylanilid)

1,8 g (6 mmol) 1H-1,2,4-Triazol-3-sulfonsäure-(2-difluormethoxy-6-methylanilid) werden in 10 ml Dimethylformamid mit 1,68 g (12 mmol) Kaliumcarbonat 10 Minuten bei 50 °C gerührt. Anschließend wird auf 10 °C abgekühlt, mit 1,33 g (6 mmol) 4-Chlor-6-methoxy-2-methylsulfonylpyrimidin versetzt und 45 Minuten bei 10 °C gerührt. Die Reaktionsmischung wird auf Eiswasser gegeben, mit 2 N Salzsäure-Lösung angesäuert (pH 4) und der Feststoff abgesaugt. Die Reinigung erfolgt durch Chromatographieren an Kieselgel mit Methylenchlorid/Methanol (95/5).

Ausbeute: 1,1 g = 41 % der Theorie
Fp.: 215 - 216 °C

Analog wurden auch die folgenden erfindungsgemäßen Verbindungen hergestellt:

| Beispiel | Name der Verbindung | Physikalische Konstante |
|---|---|---|
| 2 | 1-(4-Chlor-6-methoxypyrimidin-2-yl)-1H-1,2,4-triazol-3-sulfonsäure-(2,6-difluoranilid) | Fp.: 229-234°C |
| 3 | 1-(4-Chlor-6-methoxypyrimidin-2-yl)-1H-1,2,4-triazol-3-sulfonsäure-(2,6-dichloranilid) | Fp.: 235-240°C |
| 4 | 1-(4-Chlor-6-methoxypyrimidin-2-yl)-1H-1,2,4-triazol-3-sulfonsäure-(2-methoxycarbonyl-6-methylanilid) | Fp.: 147-152°C |
| 5 | 1-(4-Chlor-6-methoxypyrimidin-2-yl)-1H-1,2,4-triazol-3-sulfonsäure-(2,6-dichlor-3-methyl-anilid) | Fp.: 217-221°C |
| 6 | 1-(4-Chlor-6-methoxypyrimidin-2-yl)-1H-1,2,4-triazol-3-sulfonsäure-(2-chlor-6-methylanilid) | Fp.: 228-233°C |
| 7 | 1-(4-Chlor-6-ethoxy-pyrimidin-2-yl)-1H-1,2,4-triazol-3-sulfonsäure-(2,6-dichlor-3-methyl-anilid) | Fp.: 212-215°C |
| 8 | 1-(4-Chlor-6-methoxypyrimidin-2-yl)-1H-1,2,4-triazol-3-sulfonsäure-(2-trifluormethylanilid) | Fp.: 191-194°C |
| 9 | 1-(4-Chlor-6-methoxypyrimidin-2-yl)-1H-1,2,4-triazol-3-sulfonsäure-(2-methoxyanilid) | Fp.: 175-178°C |
| 10 | 1-(4-Chlor-6-ethoxy-pyrimidin-2-yl)-1H-1,2,4-triazol-3-sulfonsäure-(2-methoxycarbonyl-6-methylanilid) | Fp.: 142-148°C |

| Beispiel | Name der Verbindung | Physikalische Konstante |
|---|---|---|
| 11 | 1-(4-Chlor-6-methoxypyrimidin-2-yl)-1H-1,2,4-triazol-3-sulfonsäure-(2-methyl-6-nitroanilid) | Fp.: 221-224°C |
| 12 | 1-(4-Chlor-6-ethoxy-pyrimidin-2-yl)-1H-1,2,4-triazol-3-sulfonsäure-(2,6-dichloranilid) | Fp.: 251-259°C |
| 13 | 1-(4-Chlor-6-ethoxy-pyrimidin-2-yl)-1H-1,2,4-triazol-3-sulfonsäure-(2,6-difluoranilid) | Fp.: 190-193°C |
| 14 | 1-(4-Chlor-6-methoxypyrimidin-2-yl)-1H-1,2,4-triazol-3-sulfonsäure-(2,3-dimethyl-6-nitro-anilid) | Fp.: 226-230°C |
| 15 | 1-(4-Chlor-6-ethoxy-pyrimidin-2-yl)-1H-1,2,4-triazol-3-sulfonsäure-(2-difluormethoxy-6-methylanilid) | Fp.: 147-150°C |
| 16 | 1-(4-Chlor-6-methoxypyrimidin-2-yl)-1H-1,2,4-triazol-3-sulfonsäure-(2-difluormethoxyanilid) | Fp.: 178-181°C |
| 17 | 1-(4-Chlor-6-ethoxy-pyrimidin-2-yl)-1H-1,2,4-triazol-3-sulfonsäure-(2-difluormethoxyanilid) | Fp.: 157-161°C |
| 18 | 1-(4-Chlor-6-methoxypyrimidin-2-yl)-1H-1,2,4-triazol-3-sulfonsäure-(2,6-dibromanilid) | Fp.:266°C (Zers.) |
| 19 | 1-(4-Chlor-6-methoxypyrimidin-2-yl)-1H-1,2,4-triazol-3-sulfonsäure-(2-allyloxy-6-nitro-anilid) | Fp.: 173-176°C |
| 20 | 1-(4-Chlor-6-methoxypyrimidin-2-yl)-1H-1,2,4-triazol-3-sulfonsäure-(2-methoxy-6-nitro-anilid) | Fp.: 222-225°C |

| Beispiel | Name der Verbindung | Physikalische Konstante |
|---|---|---|
| 21 | 1-(4-Chlor-6-methoxypyrimidin-2-yl)-1H-1,2,4-triazol-3-sulfonsäure-(2-propargyloxy-6-nitro-anilid) | Fp.: 176-180°C |
| 22 | 1-(4-Chlor-6-ethoxy-pyrimidin-2-yl)-1H-1,2,4-triazol-3-sulfonsäure-(2-methoxy-6-nitro-anilid) | Fp.: 230-232°C |
| 23 | 1-(4-Chlor-6-ethoxy-pyrimidin-2-yl)-1H-1,2,4-triazol-3-sulfonsäure-(2-allyloxy-6-nitro-anilid) | Fp.: 132-135°C |
| 24 | 1-(4-Chlor-6-ethoxy-pyrimidin-2-yl)-1H-1,2,4-triazol-3-sulfonsäure-(2-propargyloxy-6-nitro-anilid) | Fp.: 192-196°C |
| 25 | 1-(4-Chlor-6-n-propoxy-pyrimidin-2-yl)-1H-1,2,4-triazol-3-sulfonsäure-(2,6-dichlor-3-methyl-anilid) | Fp.: 197-199°C |
| 26 | 1-(4-Chlor-6-n-propoxy-pyrimidin-2-yl)-1H-1,2,4-triazol-3-sulfonsäure-(2-difluormethoxy-6-methyl-anilid) | |
| 27 | 1-(4-Chlor-6-n-propoxy-pyrimidin-2-yl)-1H-1,2,4-triazol-3-sulfonsäure-(2-allyloxy-6-nitroanilid) | Fp.: 164°C |
| 28 | 1-(4-Chlor-6-n-propoxy-pyrimidin-2-yl)-1H-1,2,4-triazol-3-sulfonsäure-(2,6-difluoranilid) | |
| 29 | 1-(4-Chlor-6-n-propoxy-pyrimidin-2-yl)-1H-1,2,4-triazol-3-sulfonsäure-(2-methoxycarbonyl-6-methylanilid) | |

| Beispiel | Name der Verbindung | Physikalische Konstante |
|---|---|---|
| 30 | 1-(4-Chlor-6-n-propoxy-pyrimidin-2-yl)-1H-1,2,4-triazol-3-sulfonsäure-(2-methoxy-6-methoxycarbonylanilid) | Fp.: 166-167°C |
| 31 | 1-(4-Chlor-6-isopropoxy-pyrimidin-2-yl)-1H-1,2,4-triazol-3-sulfonsäure-(2,6-dichlor-3-methylanilid) | Fp.: 187°C |
| 32 | 1-(4-Chlor-6-isopropoxy-pyrimidin-2-yl)-1H-1,2,4-triazol-3-sulfonsäure-(2-difluormethoxy-6-methylanilid) | |
| 33 | 1-(4-Chlor-6-isopropoxy-pyrimidin-2-yl)-1H-1,2,4-triazol-3-sulfonsäure-(2-allyloxy-6-nitroanilid) | Fp.: 171-172°C |
| 34 | 1-(4-Chlor-6-isopropoxy-pyrimidin-2-yl)-1H-1,2,4-triazol-3-sulfonsäure-(2,6-difluoranilid) | |
| 35 | 1-(4-Chlor-6-isopropoxy-pyrimidin-2-yl)-1H-1,2,4-triazol-3-sulfonsäure-(2-methoxycarbonyl-6-methylanilid) | |
| 36 | 1-(4-Chlor-6-isopropoxy-pyrimidin-2-yl)-1H-1,2,4-triazol-3-sulfonsäure-(2-methoxy-6-methoxycarbonylanilid) | Fp.: 179-180°C |
| 37 | 1-(4-Chlor-6-methoxypyrimidin-2-yl)-1H-1,2,4-triazol-3-sulfonsäure-(2-methoxycarbonyl-4-methyl-3-thienylamid) | |
| 38 | 1-(4-Chlor-6-ethoxy-pyrimidin-2-yl)-1H-1,2,4-triazol-3-sulfonsäure-(2-methoxycarbonyl-4-methyl-3-thienylamid) | |

| Beispiel | Name der Verbindung | Physikalische Konstante |
|---|---|---|
| 39 | 1-(4-Chlor-6-methoxypyrimidin-2-yl)-1H-1,2,4-triazol-3-sulfonsäure-(2-methoxy-6-methoxycarbonylanilid) | Fp.: 174-176 °C |
| 40 | 1-(4-Chlor-6-methoxy-pyrimidin-2-yl)-5-methyl-1H-1,2,4-triazol-3-sulfonsäure-(2,6-difluoranilid) | Fp.: 183-185 °C |
| 41 | 1-(4-Chlor-6-methoxy-pyrimidin-2-yl)-5-methyl-1H-1,2,4-triazol-3-sulfonsäure-(2,6-dichloranilid) | Fp.: 224-225 °C |
| 42 | 1-(4-Chlor-6-methoxy-pyrimidin-2-yl)-5-methyl-1H-1,2,4-triazol-3-sulfonsäure-(2,6-dichlor-3-methylanilid) | Fp.: 214-215 °C |
| 43 | 1-(4-Chlor-6-methoxy-pyrimidin-2-yl)-5-methyl-1H-1,2,4-triazol-3-sulfonsäure-(2-methyl-6-nitroanilid) | Fp.: 209-210 °C |
| 44 | 1-(4-Chlor-6-methoxy-pyrimidin-2-yl)-5-methoxymethyl-1H-1,2,4-triazol-3-sulfonsäure-(2,6-difluoranilid) | Fp.: 185-186 °C |
| 45 | 1-(4-Chlor-6-methoxy-pyrimidin-2-yl)-5-methoxymethyl-1H-1,2,4-triazol-3-sulfonsäure-(2,6-dichloranilid) | Fp.: 193-195 °C |
| 46 | 1-(4-Chlor-6-methoxy-pyrimidin-2-yl)-5-methyl-1H-1,2,4-triazol-3-sulfonsäure-(2-difluormethoxy-6-methylanilid) | Fp.: 201-204 °C |

| Beispiel | Name der Verbindung | Physikalische Konstante |
|---|---|---|
| 47 | 1-(4-Chlor-6-methoxy-pyrimidin-2-yl)-5-methyl-1H-1,2,4-triazol-3-sulfonsäure-(2-methoxycarbonyl-6-methylanilid) | Fp.: 162-165°C |
| 48 | 1-(4-Chlor-6-methoxy-pyrimidin-2-yl)-5-methyl-1H-1,2,4-triazol-3-sulfonsäure-(2-methoxycarbonyl-4-methyl-3-thienylamid) | Fp.: 162-165°C |
| 49 | 1-(4-Chlor-6-methoxy-pyrimidin-2-yl)-5-methyl-1H-1,2,4-triazol-3-sulfonsäure-(2-chlor-6-methoxycarbonylanilid) | Fp.: 191-193°C |
| 50 | 1-(4-Chlor-6-methoxy-pyrimidin-2-yl)-5-methoxymethyl-1H-1,2,4-triazol-3-sulfonsäure-(2-difluormethoxy-6-methylanilid) | Fp.: 134-136°C |
| 51 | 1-(4-Chlor-6-methoxy-pyrimidin-2-yl)-5-methoxymethyl-1H-1,2,4-triazol-3-sulfonsäure-(2-methoxycarbonyl-4-methyl-3-thienylamid) | Fp.: 181-183°C |
| 52 | 1-(4-Chlor-6-methoxy-pyrimidin-2-yl)-5-methyl-1H-1,2,4-triazol-3-sulfonsäure-(2-methoxy-6-methoxycarbonylanilid) | Fp.: 212-213°C |

Die folgenden Beispiele erläutern die Anwendungsmöglichkeiten der erfindungsgemäßen Verbindungen:

**Beispiel A**

Im Gewächshaus wurden die aufgeführten Pflanzenspezies vor dem Auflaufen mit den aufgeführten Verbindungen in einer Aufwandmenge von 0,03 kg Wirkstoff/ha behandelt. Die Verbindungen wurden zu diesem Zweck als Emulsionen mit 500 Litern Wasser/ha gleichmäßig über die Pflanzen versprüht. Hier zeigten drei Wochen nach der Behandlung die erfindungsgemäßen Verbindungen eine hohe Kulturpflanzen-Selektivität in Weizen sowie in typischen Folgekulturen wie Zuckerrübe und Brassica sp. bei ausgezeichneter Wirkung gegen das Unkraut. Die Vergleichsmittel zeigten nicht die gleichhohe Selektivität.

In der folgenden Tabelle bedeuten:

0 = nicht geschädigt
4 = total vernichtet
- = nicht geprüft

TRZAX = Triticum aestivum
BEAVS = Beta vulgaris
BRSSS = Brassica sp.
MATCH = Matricaria chamomilla

| Erfindungsgemäße Verbindungen | TRZAX | BEAVX | BRSSS | MATCH |
|---|---|---|---|---|
| Beispiel 2 | 0 | 0 | 1 | 4 |
| Beispiel 5 | 0 | 0 | 1 | 4 |
| Beispiel 8 | 0 | 0 | 0 | 4 |
| Beispiel 9 | 0 | 0 | 0 | 4 |
| Beispiel 10 | 0 | 0 | 0 | 4 |
| Beispiel 11 | 0 | 1 | 0 | 4 |
| Beispiel 16 | 0 | 0 | 1 | 4 |
| Beispiel 17 | 0 | 0 | 0 | 4 |
| Beispiel 18 | 0 | 0 | 0 | 4 |
| Beispiel 19 | 0 | 0 | 0 | 4 |
| Beispiel 20 | 0 | 1 | 3 | 4 |
| Beispiel 21 | 0 | 1 | 2 | 4 |
| Beispiel 22 | 0 | 0 | - | 3 |
| Beispiel 23 | 0 | 0 | - | 3 |
| Beispiel 24 | 0 | 1 | - | 3 |
| Unbehandelt | 0 | 0 | 0 | 0 |
| **Vergleichsmittel** | | | | |
| Thiameturon-methyl | 0 | 3 | 3 | 4 |
| Verbindung I 30 aus EP-Anmeldung 0 246 749 | - | 3 | - | 4 |
| Verbindung I 36 aus EP-Anmeldung 0 246 749 | - | 4 | - | 4 |

**Beispiel B**

Im Gewächshaus wurden die aufgeführten Pflanzenspezies nach dem Auflaufen mit den aufgeführten Verbindungen in einer Aufwandmenge von 0,03 kg Wirkstoff/ha behandelt. Die Verbindungen wurden zu

diesem Zweck als Emulsionen mit 500 Litern Wasser/ha gleichmäßig über die Pflanzen versprüht. Hier zeigten zwei Wochen nach der Behandlung die erfindungsgemäßen Verbindungen eine hohe Kulturpflanzen-Selektivität in Weizen sowie in typischen Folgekulturen wie Zuckerrübe und Brassica sp. sowie in Baumwolle bei ausgezeichneter Wirkung gegen das Unkraut. Das Vergleichsmittel zeigte nicht die gleichhohe Selektivität.

In der folgenden Tabelle bedeuten:

0 = nicht geschädigt

4 = total vernichtet

\- = nicht geprüft

TRZAX = Triticum aestivum

BEAVS = Beta vulgaris

BRSSS = Brassica sp.

GOSHI = Gossypium hirsutum

MATCH = Matricaria chamomilla

| Erfindungsgemäße Verbindungen | TRZAX | BEAVX | BRSSS | GOSHI | MATCH |
|---|---|---|---|---|---|
| Beispiel 2 | 0 | 0 | 1 | 0 | 3 |
| Beispiel 3 | 0 | 1 | 1 | 0 | 3 |
| Beispiel 4 | 0 | 1 | 0 | 1 | 3 |
| Beispiel 5 | 0 | 0 | 0 | 0 | 3 |
| Beispiel 6 | 0 | 1 | 1 | 0 | 4 |
| Beispiel 7 | 0 | 0 | 0 | 0 | 3 |
| Beispiel 8 | 0 | 0 | 0 | 0 | 4 |
| Beispiel 10 | 0 | 0 | 0 | 0 | 3 |
| Beispiel 11 | 0 | 0 | 0 | 0 | 4 |
| Beispiel 15 | 0 | 0 | - | 0 | 4 |
| Beispiel 16 | 0 | 0 | 1 | 0 | 4 |
| Beispiel 20 | 0 | 1 | 3 | 0 | 3 |
| Beispiel 21 | 0 | 0 | 2 | 0 | 3 |
| Beispiel 22 | 0 | 0 | - | 0 | 3 |
| Beispiel 23 | 0 | 0 | - | 0 | 3 |
| Beispiel 24 | 0 | 0 | - | 0 | 3 |
| Unbehandelt | 0 | 0 | 0 | 0 | 0 |
| Vergleichsmittel | | | | | |
| Thiameturon-methyl | 0 | 4 | 4 | 3 | 4 |

**Beispiel C**

Die erfindungsgemäßen Verbindungen wurden unter Gewächshausbedingungen im Vorauflaufverfahren auf Töpfe mit Matricaria verschiedener Herkunft beziehungsweise Beta-Rübenvarietäten gespritzt. Die verwendete Wassermenge entsprach ca. 200 Liter/ha. Nachfolgend ist der Pflanzenwuchs als Relativwert des Frischgewichtes zu Unbehandelt vier Wochen nach der Behandlung angegeben.

Wie das Beispiel zeigt, sind die erfindungsgemäßen Substanzen gut rübenverträglich bei gleichzeitig sehr guter Wirkung gegen Kamille.

In der folgenden Tabelle bedeuten:

MATCH = Matricaria chamomilla

BEAVA = Beta vulgaris ssp. vulgaris var. altissima

BEAVC = Beta vulgaris ssp. vulgaris var. crassa

| Erfindungsgemäße Verbindungen | Dosis g Wirkstoff/ha | Frischgewicht (relativ zu Unbehandelt) MATCH | MATCH (UK) | BEAVA | BEAVC |
|---|---|---|---|---|---|
| Beispiel 3 | 3 | 9 | 6 | 98 | 98 |
| | 10 | 0 | 0 | 104 | 99 |
| Beispiel 5 | 10 | 6 | 2 | 96 | 97 |
| | 30 | 0 | 0 | 101 | 95 |
| Beispiel 8 | 10 | 0 | 0 | 99 | 99 |
| | 30 | 0 | 0 | 93 | 97 |
| | 100 | 0 | 0 | 98 | 192 |
| Beispiel 19 | 30 | 0 | 0 | 104 | 97 |
| | 100 | 0 | 0 | 99 | 99 |
| Beispiel 20 | 10 | 0 | 0 | 100 | 103 |
| Beispiel 21 | 10 | 0 | 0 | 110 | 106 |
| | 30 | 0 | 0 | 94 | 92 |
| Beispiel 22 | 10 | 0 | 0 | 98 | - |
| | 30 | 0 | 0 | 97 | - |
| Beispiel 23 | 10 | 1 | 1 | 69 | 104 |
| | 30 | 0 | 0 | 100 | 107 |
| Beispiel 24 | 10 | 0 | 0 | 94 | 107 |
| | 30 | 0 | 0 | 93 | 110 |

| Erfindungsgemäße Verbindungen | Dosis g Wirkstoff/ha | Frischgewicht (relativ zu Unbehandelt) | | | |
|---|---|---|---|---|---|
| | | MATCH | MATCH (UK) | BEAVA | BEAVC |
| Unbehandelt | - | 100 | 100 | 100 | 100 |
| Vergleichsmittel | | | | | |
| Ethofumesate | 500 | 107 | 78 | 89 | 96 |
| | 1000 | 88 | 82 | 74 | 100 |

**Beispiel D**

Die erfindungsgemäßen Verbindungen wurden unter Gewächshausbedingungen im Nachauflaufverfahren auf Töpfe mit Pflanzen von Matricaria-Arten beziehungsweise Beta-Rübenvarietäten appliziert. Die verwendete Wassermenge entsprach 200 Liter/ha. Zwei Wochen nach der Behandlung wurde das Frischgewicht der Pflanzen bestimmt, das in nachfolgender Tabelle als Relativwert zu Unbehandelt angegeben ist.

Wie das Beispiel zeigt, sind die erfindungsgemäßen Substanzen gut rübenverträglich bei gleichzeitig sehr guter Wirkung gegen Kamille (das Vergleichsmittel erreicht die gute Wirkung nicht).

In der folgenden Tabelle bedeuten:

MATCH = Matricaria chamomilla
MATIN = Matricaria inodora
MATMT = Matricaria matricarioides
BEAVA = Beta vulgaris ssp. vulgaris var. altissima
BEAVC = Beta vulgaris ssp. vulgaris var. crassa
BEAVD = Beta vulgaris ssp. vulgaris var. conditiva

| Erfindungsgemäße Verbindungen | Dosis g Wirkstoff/ha | Frischgewicht (relativ zu Unbehandelt) MATCH | MATCH (UK) | MATIN | MATMT | BEAVA | BEAVC | BEAVD |
|---|---|---|---|---|---|---|---|---|
| Beispiel 3 | 300 | 4 | 7 | 19 | 3 | 93 | 91 | 85 |
| Beispiel 4 | 100 | 6 | 7 | 21 | 18 | 98 | 95 | 86 |
| | 300 | 3 | 5 | 9 | 7 | 95 | 95 | 81 |
| Beispiel 5 | 300 | 12 | 13 | 54 | 26 | 106 | 99 | 90 |
| Beispiel 6 | 300 | 6 | 6 | 8 | 14 | 95 | 94 | 99 |
| Beispiel 8 | 100 | 6 | 11 | 48 | 13 | 107 | 103 | 98 |
| Beispiel 22 | 300 | 8 | 4 | 13 | 7 | 91 | 95 | 98 |
| Beispiel 23 | 300 | 9 | 10 | 20 | 12 | 95 | 96 | 95 |
| Beispiel 24 | 300 | 8 | 9 | 16 | 12 | 99 | 96 | 110 |
| Unbehandelt | - | 100 | 100 | 100 | 100 | 100 | 100 | 100 |
| Vergleichsmittel | | | | | | | | |
| Phenmedipham | 320 | 46 | 49 | 50 | 26 | 104 | 103 | 105 |
| | 960 | 18 | 23 | 20 | 12 | 98 | 93 | 90 |

**Beispiel E**

Im Gewächshaus wurden die aufgeführten Pflanzenspezies vor dem Auflaufen mit den aufgeführten erfindungsgemäßen Verbindungen in der angegebenen Aufwandmenge behandelt. Die erfindungsgemäßen Verbindungen wurden als Aceton-Lösungen, die ein Netzmittel enthielten, gleichmäßig über die Gefäße, die Samen der Pflanzenarten enthielten, versprüht. Nach 3 bis 4 Wochen wurden die Pflanzen bezüglich einer herbizider Wirkung beurteilt.

In der folgenden Tabelle bedeuten:

0 = keine Wirkung
1 = 1 - 24 % Wirkung
2 = 25 - 69 % Wirkung
3 = 70 - 89 % Wirkung
4 = 90 - 100 % Wirkung
- = nicht geprüft

GALAP = Galium aparine
MATIN = Matricaria inodora
MATCH = Matricaria chamomila
BEAVS = Beta vulgaris

| Erfindungsgemäße Verbindungen | kg Wirkstoff/ha | GALAP | MATIN | MATCH | BEAVS |
|---|---|---|---|---|---|
| Beispiel 40 | 0,032 | 3 | 4 | - | 0 |
| Beispiel 41 | 0,25 | 3 | - | - | 2 |
| Beispiel 42 | 0,016 | 1 | 4 | 4 | 0 |
| Beispiel 43 | 0,25 | 3 | - | - | 2 |
| Beispiel 44 | 0,25 | 2 | - | - | 2 |
| Beispiel 45 | 0,25 | 3 | - | - | 2 |
| Beispiel 46 | 0,25 | 3 | - | - | 2 |
| Beispiel 47 | 0,016 | 3 | 4 | 4 | 0 |
| Beispiel 48 | 0,25 | 2 | 4 | 2 | 1 |
| Beispiel 49 | 0,016 | 2 | 4 | 4 | 1 |
| Beispiel 50 | 0,016 | - | 4 | 4 | 1 |
| Beispiel 51 | 0,032 | 2 | 4 | - | 2 |
| Beispiel 52 | 0,25 | 4 | - | - | - |
| Unbehandelt | - | 0 | 0 | 0 | 0 |

**Beispiel F**

Im Gewächshaus wurden die aufgeführten Pflanzenspezies nach dem Auflaufen mit den aufgeführten erfindungsgemäßen Verbindungen in der angegebenen Aufwandmenge behandelt. Die erfindungsgemäßen Verbindungen wurden als Aceton-Lösungen, die ein Netzmittel enthielten, gleichmäßig über die Gefäße, die Sämlinge der Pflanzenarten enthielten, versprüht. Nach 3 bis 4 Wochen wurden die Pflanzen bezüglich einer herbizider Wirkung beurteilt.

In der folgenden Tabelle bedeuten:

0 = keine Wirkung

1 = 1 - 24 % Wirkung

2 = 25 - 69 % Wirkung

3 = 70 - 89 % Wirkung

4 = 90 - 100 % Wirkung

- = nicht geprüft

GALAP = Galium aparine

MATIN = Matricaria inodora

MATCH = Matricaria chamomila

BEAVS = Beta vulgaris

| Erfindungsgemäße Verbindungen | kg Wirkstoff/ha | GALAP | MATIN | MATCH | BEAVS |
|---|---|---|---|---|---|
| Beispiel 40 | 0,016 | 3 | 4 | 4 | 2 |
| Beispiel 41 | 0,25 | 4 | - | - | 2 |
| Beispiel 42 | 0,125 | 3 | - | - | 1 |
| Beispiel 43 | 0,25 | 2 | - | - | 2 |
| Beispiel 44 | 0,25 | 2 | - | - | 2 |
| Beispiel 45 | 0,016 | 2 | 4 | 4 | 0 |
| Beispiel 46 | 0,25 | 4 | 3 | 3 | 2 |
| Beispiel 47 | 0,25 | 3 | - | - | - |
| Beispiel 49 | 0,125 | 4 | - | - | 2 |
| Beispiel 50 | 0,125 | 4 | 4 | - | 2 |
| Beispiel 52 | 0,125 | 4 | 4 | - | 2 |
| Unbehandelt | - | 0 | 0 | 0 | 0 |

## Patentansprüche

**1.** 1-Chlorpyrimidinyl-1H-1,2,4-triazol-3-sulfonsäureamide der allgemeinen Formel I

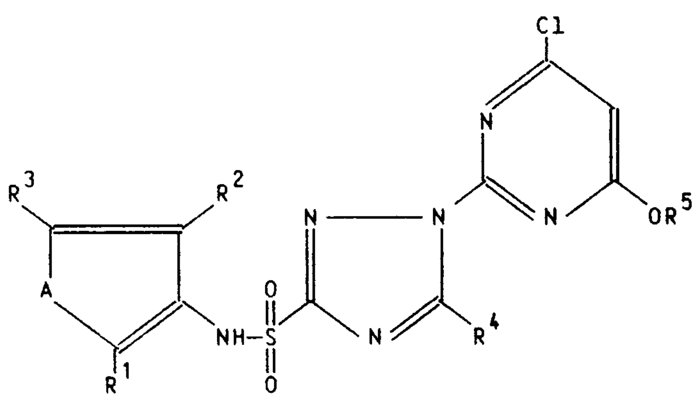

(I) ,

in der

A -S- oder -CH=CH-

$R^1$ Halogen, Methyl, Trifluormethyl, Methoxy, Difluormethoxy, Nitro oder Methoxycarbonyl,

$R^2$ Wasserstoff, Halogen, Methyl, Methoxy, Allyloxy oder Propargyloxy,

$R^3$ Wasserstoff oder Methyl,

$R^4$ Wasserstoff, Methyl oder Methoxymethyl und

$R^5$ $C_1$-$C_4$-Alkyl

bedeuten.

**2.** Verfahren zur Herstellung von Verbindungen der allgemeinen Formel I, dadurch gekennzeichnet, daß man

A) Amine der allgemeinen Formel II

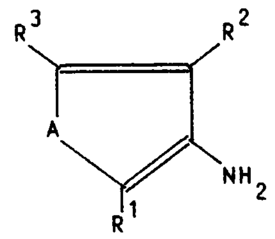

(II) ,

in der A, $R^1$, $R^2$ und $R^3$ die oben angegebenen Bedeutungen haben, mit einem Sulfonsäurechlorid der allgemeinen Formel III

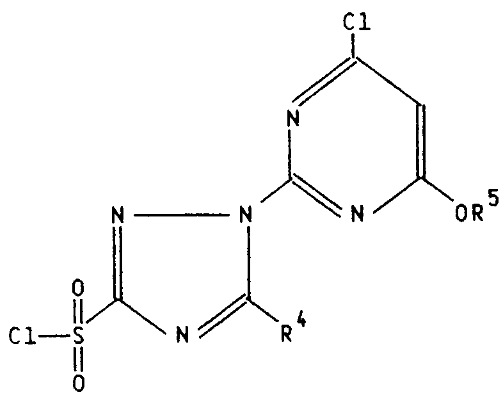

(III) ,

in der $R^4$ und $R^5$ die oben angegebenen Bedeutungen haben, in einem geeigneten Lösungsmittel in Gegenwart eines Säureakzeptors umsetzt, oder

B) eine Verbindung der allgemeinen Formel IV

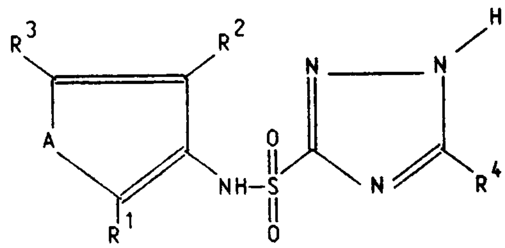

(IV),

in der A, $R^1$, $R^2$, $R^3$ und $R^4$ die oben genannten Bedeutungen haben, mit einer Verbindung der allgemeinen Formel V

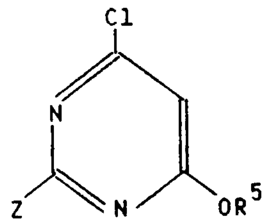

(V) ,

in der Z für Chlor, Brom oder eine Alkyl- oder Arylsulfonylgruppe steht und $R^5$ die oben genannte Bedeutung hat, in einem geeigneten Lösungsmittel in Gegenwart eines säurebindenden Mittels umsetzt.

**3.** Mittel mit herbizider Wirkung, gekennzeichnet durch einen Gehalt an mindestens einer Verbindung gemaß Anspruch 1.

**4.** Verwendung von Mitteln gemäß dem Anspruchen 3 zur Bekämpfung monokotyler und dikotyler Unkrautarten in landwirtschaftlichen Hauptkulturen.

**5.** Verfahren zur Herstellung von Mitteln mit herbizider Wirkung, dadurch gekennzeichnet, daß man Verbindungen der allgemeinen Formel I gemäß Anspruch 1 mit Träger- und/oder Hilfsstoffen vermischt.

**Claims**

**1.** 1-Chloropyrimidinyl-1H-1,2,4-triazole-3-sulphonamides of general formula I

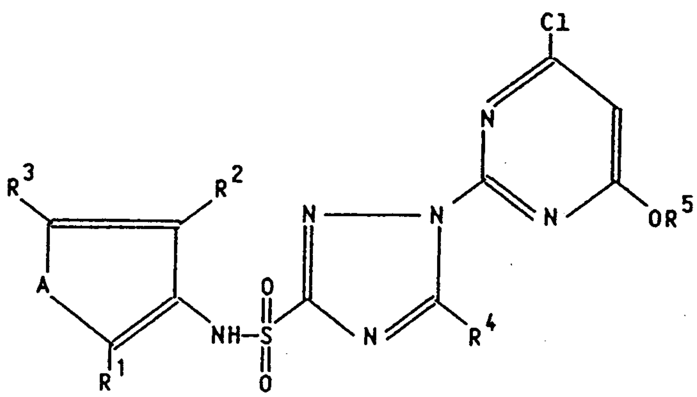

(I) ,

in which

A is -S- or -CH = CH-;
$R^1$ is halogen, methyl, trifluoromethyl, methoxy, difluoromethoxy, nitro or methoxycarbonyl;
$R^2$ is hydrogen, halogen, methyl, methoxy, allyloxy or propargyloxy;
$R^3$ is hydrogen or methyl;
$R^4$ is hydrogen, methyl or methoxymethyl; and
$R^5$ is $C_1$-$C_4$-alkyl.

**2.** Process for the preparation of compounds of general formula I, characterised by

A) reacting an amine of general formula II

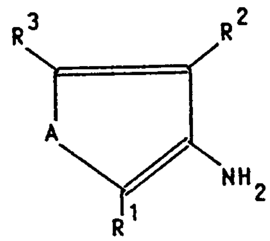

(II) ,

in which A, $R^1$, $R^2$ and $R^3$ have the meanings given above, with a sulphonylchloride of general formula III

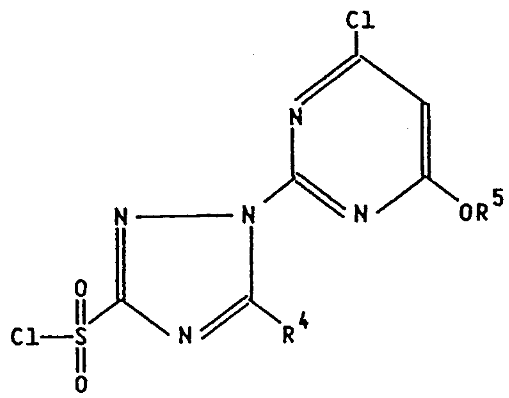

(III) ,

in which $R^4$ and $R^5$ have the meanings given above, in a suitable solvent and in the presence of an acid acceptor, or

B) reacting a compound of general formula IV

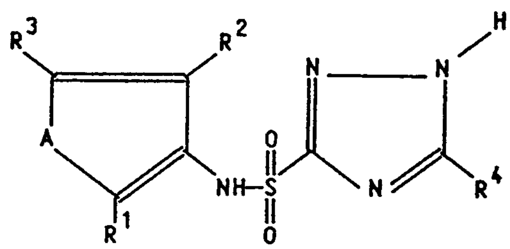

(IV),

in which A, $R^1$, $R^2$, $R^3$ and $R^4$ have the meanings given above, with a compound of general formula V

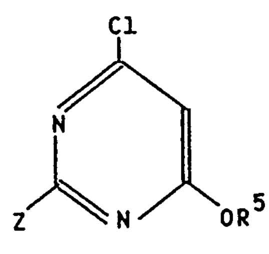

(V) ,

in which Z is chlorine, bromine or an alkyl- or arylsulphonyl group and $R^5$ has the meaning given above, in a suitable solvent in the presence of an acid-binding agent.

**3.** Herbicidal composition characterised by a content of at least one compound according to claim 1.

**4.** Use of compositions according to claim 3 for combating monocotyledonous and dicotyledonous weeds in agricultural main cultures.

**5.** Process for the preparation of compositions, having herbicidal activity, characterised by mixing compounds of general formula I, according to claim 1, with carriers and/or diluents.

**Revendications**

**1.** 1-Chloropyrimidinyl-1H-1,2,4-triazole-3-sulfonamides de formule générale I ci-dessous

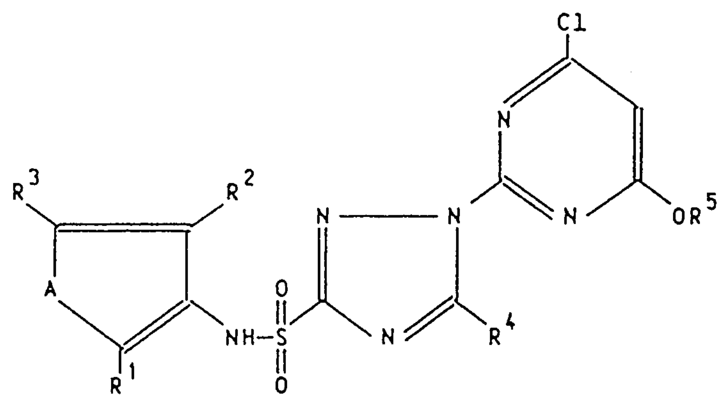

(I)

dans laquelle

- A désigne -S- ou -CH=CH-
- $R^1$ un halogène ou un groupe méthyle, trifluorométhyle, méthoxy, difluorométhoxy, nitro ou méthoxycarbonyle,
- $R^2$ l'hydrogène, un halogène ou un groupe méthyle, méthoxy, allyloxy ou propargyloxy,
- $R^3$ l'hydrogène ou le groupe méthyle,
- $R^4$ l'hydrogène ou un groupe méthyle ouméthoxyméthyle et
- $R^5$ un alkyle en $C_1$-$C_4$.

**2.** Procédé de préparation des composés de formule I selon la revendication 1, procédé caractérisé en ce que :

A) on fait réagir des amines de formule générale II

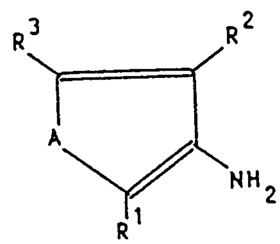

(II)

les divers symboles ayant les significations indiquées à la revendication 1, avec un chlorure d'acide sulfonique de formule générale III

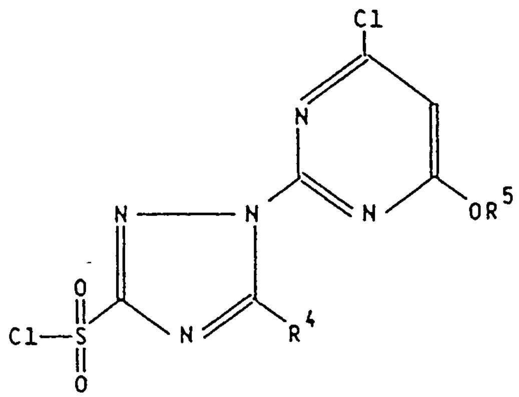

(III)

$R^4$ et $R^5$ ayant les significations données à la revendiction 1, dans un solvant approprié et en présence d'un accepteur d'acides, ou bien
B) on fait réagir un composé de formule générale IV

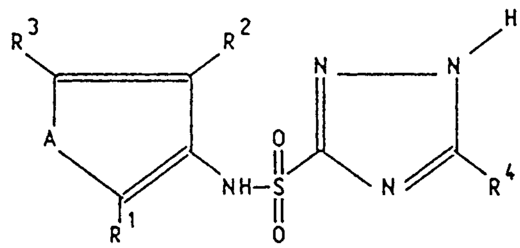

(IV)

les divers symboles ayant les significations données à la revendication 1, avec un composé de formule générale V

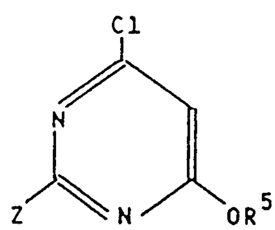

(V)

Z désignant le chlore, le brome ou un groupe alkyl- ou aryl-sulfonyle et $R^5$ ayant la signification donnée à la revendication 1, dans un solvant approprié et en présence d'un agent liant les acides.

**3.** Produit herbicide caractérisé en ce qu'il comprend un ou plusieurs composés selon la revendication 1.

**4.** L'emploi de produits selon la revendication 3 pour lutter contre des sortes de mauvaises herbes ou plantes adventices monocotylédones et dicotylédones en agriculture.

**5.** Procédé de préparation de produits herbicides caractérisé en ce que l'on mélange des composés de formule I selon la revendication 1 avec des véhicules et/ou des adjuvants.